# EUROPÄISCHE PATENTSCHRIFT

(11) **EP 4 121 199 B1**
(45) Veröffentlichungstag und Bekanntmachung des Hinweises auf die Patenterteilung: **21.10.2026**
(21) Anmeldenummer: 21709984.5
(22) Anmeldetag: 04.03.2021
(51) Int. Cl.: B01D 63/02, A61M 1/16

(54) **DIALYSATOR, DIALYSEGERÄT SOWIE KIT UND VERFAHREN ZUM HERSTELLEN EINES DIALYSATORS**
DIALYSER, DIALYSIS EQUIPMENT AND KIT AND METHOD FOR PRODUCING A DIALYSER
DIALYSEUR, ÉQUIPEMENT DE DIALYSE ET KIT ET PROCÉDÉ DE FABRICATION D'UN DIALYSEUR

(30) Priorität: 19.03.2020 DE 102020203553
(43) Veröffentlichungstag der Anmeldung: 25.01.2023
(73) Patentinhaber: B. Braun Avitum AG, 34212 Melsungen (DE)
(72) Erfinder: MANDRY, Peter, 01326 Dresden (DE)
(74) Vertreter: Patentanwälte Ruff, Wilhelm, Beier, Dauster & Partner mbB
(86) Internationale Anmeldenummer: PCT/EP2021/055476
(87) Internationale Veröffentlichungsnummer: WO 2021/185591

(56) Entgegenhaltungen:
- WO-A1-2008/025459
- WO-A1-2013/095682
- DE-A1- 19 806 293
- DE-T1- 19 782 098
- GB-A- 2 059 290
- JP-A- H08 168 525
- US-A- 4 276 687

## Beschreibung

Die Erfindung betrifft einen Dialysator, ein Dialysegerät sowie ein Kit und ein Verfahren zum Herstellen eines Dialysators.

Hohlfasern kommen als semipermeable Membranen (sogenannte Hohlfasermembranen) unter anderem bei der Herstellung von Dialysatoren zum Einsatz. Dialysatoren sind Filtermodule für die extrakorporale Blutbehandlung, wie beispielsweise Hämodialyse, Hämofiltration sowie Hämodiafiltration. Derartige Hohlfasern können auch als Membranfasern bezeichnet werden. Dialysatoren stellen das Herzstück eines Dialysegerätes dar. Üblicherweise weisen Dialysatoren ein röhrenförmig gestaltetes Dialysatorgehäuse mit einer Längsausdehnung auf. Im Innenraum des Dialysators sind parallel zueinander angeordnete Hohl- bzw. Membranfasern angeordnet. Das zu behandelnde bzw. zu reinigende Blut fließt dabei durch das Innere der Hohlfasern, während der Außenraum der Hohlfasern sowie der Innenraum des Dialysatorgehäuses gegenläufig von einem Dialysat durchströmt wird. Durch die semipermeablen Membranen der Hohlfasern finden sowohl ein Wasser- als auch ein Stoffaustausch statt. Insbesondere werden dem Blut eines Patienten Wasser und Schadstoffe entzogen.

Zur Herstellung der Dialysatoren werden die Hohlfasern in Form von Bündeln abgelegt und mit einer Hüllfolie umwickelt. Die Hüllfolie besitzt zweierlei Funktionen. Zum einen hält sie die Bündelform der Hohlfasern aufrecht. Zum anderen schützt die Hüllfolie die äußeren Hohlfasern des Bündels vor Beschädigungen während des Herstellungsprozesses, vor allem während des Einführens des umwickelten Hohlfaserbündels in ein Dialysatorgehäuse. Bei derzeit üblichen Verfahren zur Herstellung von Dialysatoren muss nach dem Einführen des umwickelten Hohlfaserbündels in das Dialysatorgehäuse die Hüllfolie aus diesem entfernt werden. Anderenfalls würde die Hüllfolie zum einen die Verteilung einer zur Herstellung einer Hohlfasereinbettung verwendeten Vergussmasse beim Vergießen der Hohlfasern stören. Zum anderen würde die Hüllfolie bei Inbetriebnahme des Dialysators den Dialysatfluss und mithin eine gleichmäßige Umströmung der Hohlfasern behindern. Entsprechende Dialysatoren sind beispielsweise aus den Druckschriften DE 197 82 098 T2, JP 4069998 B2 und EP 2 056 956 B1 bekannt. JP-H08-168525A offenbart eine Schutzhülle um das Hohlfasermoembranmodul eines Dialysators aus Nylon und Polyurethanfäden. In WO2013/095682A1 sind die Hohlfasern von einem Netz umgeben.

Nachteilig ist, dass beim Herausziehen der Hüllfolie aus dem Dialysatorgehäuse durch Umknicken einzelner Hohlfasern sogenannte Knickfasern entstehen können. Diese Fasern können nicht an ihren axialen Endabschnitten mit der Vergussmasse, beispielsweise Polyurethan, vergossen werden und haben daher ihre Öffnung nicht im extrakorporalen Blutkreislauf, sondern im Kreislauf der Dialysierflüssigkeit. Die Folge ist, dass beim bestimmungsgemäßen Gebrauch des Dialysators ein Blutleck entsteht, d.h. Blut in den Dialysatkreislauf eines Dialysegeräts fließt. Daher müssen Dialysatoren vor ihrer Auslieferung standardmäßig im Rahmen eines Integritätstests auf das Vorliegen eines solchen Defekts geprüft werden. Die hiervon betroffenen Dialysatoren stellen einen unerwünschten Ausschuss dar. Das Vorliegen von Knickfasern ist ursächlich für etwa 2% bis 5% des Ausschusses bei Dialysatoren. Damit nehmen Knickfasern als Ausschussursache eine unter Produktionsgesichtspunkten signifikante Größenordnung ein.

Das Phänomen der Knickfasern nimmt mit der Packungsdichte, d.h. der Anzahl an Hohlfasern, welche von der Hüllfolie umwickelt sind, zu. Mit zunehmender Packungsdichte üben die Hohlfasern von innen einen zunehmend höheren Druck auf die Hüllfolie aus. Dadurch wird die Hüllfolie nach dem Einführen des umwickelten Hohlfaserbündels in das Dialysatorgehäuse zwischen Gehäuse des Dialysators und Hohlfasern eingeklemmt. Mit zunehmendem Druck steigt jedoch das Risiko, dass beim Herausziehen der Hüllfolie zunehmend mehr Fasern mitgenommen und umgeknickt werden. Dies wiederum hat zur Folge, dass die prinzipiell erreichbare Packungsdichte der Hohlfasern durch das Risiko des Auftretens eines knickfaserbedingten Ausschusses begrenzt wird. Eine hohe Packungsdichte der Hohlfasern hat jedoch für den Anwender große Vorteile. So ist aufgrund einer höheren Packungsdichte der Hohlfasern eine einfachere Entlüftung des Dialysators ohne Drehung möglich.

### AUFGABE UND LÖSUNG

Aufgabe der Erfindung ist es daher, einen Dialysator bereitzustellen, welcher die eingangs, im Zusammenhang konventioneller Dialysatoren genannten Nachteile vermeidet, insbesondere das Risiko eines knickfaserbedingten Ausschusses reduziert oder sogar vollständig beseitigt. Ferner ist es Aufgabe der Erfindung, ein Dialysegerät sowie ein Kit und ein Verfahren zum Herstellen eines Dialysators bereitzustellen.

Die vorgenannten Aufgaben werden erfindungsgemäß gelöst durch einen Dialysator mit den Merkmalen des unabhängigen Anspruchs 1, ein Dialysegerät gemäß Anspruch 11 ein Kit gemäß Anspruch 12 sowie ein Verfahren gemäß Anspruch 13 Bevorzugte Ausgestaltungen der Erfindung sind in den abhängigen Ansprüchen definiert. Der Wortlaut sämtlicher Ansprüche wird hiermit durch ausdrückliche Bezugnahme zum Inhalt der vorliegenden Beschreibung gemacht.

Gemäß einem ersten Aspekt betrifft die Erfindung einen Dialysator.

Der Dialysator weist eine Vielzahl von Hohlfasern auf. Die Holfasern sind semipermeabel gestaltet, d.h. es ist sowohl ein Wasser- als auch ein Stoffaustausch über die Wandungen der Hohlfasern möglich. Typischerweise handelt es sich bei den Hohlfasern um polymere Hohlfasern. Beispielsweise können die Hohlfasern aus Polysulfon gebildet sein. Die Hohlfasern können eine Länge von 5 cm bis 200 cm, insbesondere 10 cm bis 100 cm, bevorzugt 15 cm bis 45 cm, aufweisen. Ferner können die Hohlfasern eine Wandungsdicke von 10 µm bis 200 µm, insbesondere 15 µm bis 50 µm, bevorzugt 20 µm bis 40 µm, aufweisen. Ferner können die Hohlfasern einen Innendurchmesser von 50 µm bis 2 mm, insbesondere 100 µm bis 500 µm, bevorzugt 150 µm bis 250 µm, aufweisen. Ferner können die Hohlfasern einen Außendurchmesser von 60 µm bis 3 mm, insbesondere 100 µm bis 1 mm, bevorzugt 160 µm bis 300 µm, aufweisen.

Die Vielzahl von Hohlfasern ist bzw. die Hohlfasern sind in einem Gehäuse, d.h. in einem Innenraum eines Gehäuses, des Dialysators angeordnet. Die Vielzahl von Hohlfasern ist bzw. die Hohlfasern sind zweckmäßigerweise in Längsrichtung des Dialysatorgehäuses erstreckt und insbesondere quer zur Längsrichtung des Dialysatorgehäuses nebeneinander angeordnet. Bevorzugt sind die Hohlfasern parallel oder im Wesentlichen parallel zueinander angeordnet. Das Gehäuse des Dialysators ist üblicherweise rohrförmig gestaltet. Grundsätzlich kann das Gehäuse einen kreisförmigen, elliptischen, ovalen oder polygonen, beispielsweise dreieckigen, rechteckigen, quadratischen, fünfeckigen oder sechseckigen, Querschnitt, besitzen. Bevorzugt ist das Gehäuse des Dialysators rundrohrförmig, d.h. als Rohr mit einem kreisförmigen Querschnitt, gestaltet.

Der Dialysator gemäß der vorliegenden Erfindung zeichnet sich besonders dadurch aus, dass die Vielzahl von Hohlfasern bzw. die Hohlfasern in dem Gehäuse des Dialysators mit einem textilen Flächengebilde umwickelt ist bzw. sind, wobei das textile Flächengebilde flüssigkeitsdurchlässig ist. Insbesondere kann das textile Flächengebilde wasser- und/oder dialysatdurchlässig, und/oder wenigstens teilweise, insbesondere nur teilweise oder vollständig, wasserlöslich gestaltet sein.

Durch das textile Flächengebilde sind die Hohlfasern bevorzugt bündelförmig, d.h. in Form eines Hohlfaserbündels, zusammengefasst. Das Hohlfaserbündel kann einen kreisförmigen, elliptischen, ovalen oder polygonen, beispielsweise dreieckigen, rechteckigen, quadratischen, fünfeckigen oder sechseckigen, Querschnitt besitzen. Bevorzugt besitzt das Hohlfaserbündel einen kreisförmigen Querschnitt.

Das um die Hohlfasern bzw. das Hohlfaserbündel gewickelte textile Flächengebilde kann im Sinne der vorliegenden Erfindung auch als textile Wickelhülle oder textiles Wickelgebilde bezeichnet werden. Dabei können die Hohlfasern bzw. kann das Hohlfaserbündel ein- oder mehrlagig, insbesondere wenigstens abschnittsweise, bevorzugt nur abschnittsweise, dreilagig, von dem textilen Flächengebilde umwickelt sein.

Unter dem im Zusammenhang des textilen Flächengebildes verwendeten Ausdruck "dialysatdurchlässig" soll im Sinne der vorliegen Erfindung ein textiles Flächengebilde verstanden werden, welches für ein Dialysat durchlässig ist.

Unter dem Ausdruck "Dialysat" soll im Sinne der vorliegenden Erfindung eine wässrige Lösung verstanden werden, welche in der Nierenersatztherapie, insbesondere bei der Hämodialyse, verwendet wird, um gelöste Substanzen aus dem Blut aufzunehmen und/oder in dieses abzugeben. Das Dialysat weist insbesondere Elektrolyte, Glucose sowie Puffer in einer dem Blutserum grundsätzlich entsprechenden Zusammensetzung auf.

Die Erfindung zeichnet sich insbesondere durch die nachfolgenden Vorteile aus:
- Dadurch, dass das textile Flächengebilde flüssigkeitsdurchlässig und/oder wenigstens teilweise wasserlöslich gestaltet ist, bietet es einem Dialysatfluss während eines bestimmungsgemäßen Gebrauchs des Dialysators und einer Vergussmasse zur Herstellung einer Hohlfasereinbettung während der Herstellung des Dialysators keinen nennenswerten Widerstand.

Das textile Flächengebilde kann somit im Dialysator verbleiben bzw. kann - im Falle eines wenigstens teilweise wasserlöslich gestalteten textilen Flächengebildes - wenigstens zunächst im Dialysator verbleiben. Ein Entfernen oder Herausziehen des textilen Flächengebildes ist entbehrlich. Beschädigungen der Hohlfasern, insbesondere in Form von Knickfasern, wie sie bei der Herstellung konventioneller Dialysatoren auftreten, können beträchtlich reduziert oder sogar vollständig vermieden werden. Dies wiederum bewirkt eine signifikante Senkung der Ausschussrate.
- Durch die Senkung der Ausschussrate können die Herstellungskosten des Dialysators spürbar reduziert werden.
- Ist das textile Flächengebilde wasserlöslich gestaltet, kann es nach dem Einführen der mit dem textilen Flächengebilde umwickelten Hohlfasern in ein Dialysatorgeäuse bei der Dichtigkeitsüberprüfung der Hohlfasern (mittels des sogenannten Bubble-Tests), bei welcher Dialysatoren mit Wasser gespült werden, gelöst und aus dem Dialysatorgehäuse herausgespült werden.
- Dadurch, dass das textile Flächengebilde im Dialysator verbleiben und/oder wenigstens teilweise wasserlöslich sein kann, ist eine Entsorgung des textilen Flächengebildes nicht erforderlich.
- Ferner ist von Vorteil, dass eine höhere Packungsdichte der Hohlfasern und mithin insbesondere ein größerer Durchmesser des Hohlfaserbündels gewählt werden kann, als dies bei konventionellen Dialysatoren der Fall ist. Dadurch vereinfacht sich die Handhabung des erfindungsgemäßen Dialysators deutlich. So erlaubt eine höhere Packungsdichte insbesondere eine drehfreie und mithin einfachere Entlüftung des Dialysators.
- Schließlich besteht ein weiterer Vorteil darin, dass das textile Flächengebilde gezielt funktionalisiert werden kann, wodurch das textile Flächengebilde zusätzliche Aufgaben, wie beispielsweise die Aufgabe eines DNA- und/oder Endotoxin- und/oder Bakterien- und/oder Virenadsorbers, übernehmen kann.

In Ausgestaltung der Erfindung weist das textile Flächengebilde einen Porendurchmesser, insbesondere mittleren Porendurchmesser, von 0,1 µm bis 10 mm, insbesondere 200 µm bis 1 mm, vorzugsweise 1 mm bis 5 mm, auf. Der Porendurchmesser, insbesondere mittlere Porendurchmesser, des textilen Flächengebildes ist vorzugsweise mittels Quecksilberporosimetrie bestimmt. Die in diesem Absatz offenbarten Porendurchmesser haben sich unter Flüssigkeitsdurchlässigkeitsgesichtspunkten des textilen Flächengebildes als besonders vorteilhaft herausgestellt.

In weiterer Ausgestaltung der Erfindung ist das textile Flächengebilde als Vlies oder Vliesstoff, bevorzugt als Vliesstoff, gestaltet.

Unter dem Ausdruck "Vliesstoff" soll im Sinne der vorliegenden Erfindung ein Gebilde aus Fasern, insbesondere Spinnfasern (Stapelfasern) und/oder Endlosfasern (Filamenten), und/oder Garnen, insbesondere geschnittenen Garnen, jeglicher Art und jeglichen Ursprungs verstanden werden, welche auf irgendeine Weise zu einem Vlies (einer Faserschicht oder einem Faserflor) zusammengefügt und auf irgendeine Weise miteinander verbunden worden sind, wobei das Verkreuzen bzw. Verschlingen von Fasern oder Garnen, wie es beim Weben, Wirken, Stricken, der Spitzenherstellung, dem Flechten und der Herstellung von getufteten Erzeugnissen geschieht, ausgenommen ist.

Unter dem Ausdruck "Garne" sollen im Sinne der vorliegenden Erfindung linienförmige textile Gebilde aus zwei oder mehr Fasern verstanden werden.

Das Vlies oder der Vliesstoff kann Fasern oder Garne mit einem Durchmesser von 1 µm bis 1 mm, insbesondere 10 µm bis 100 µm, vorzugsweise 20 µm bis 50 µm, aufweisen.

Ferner kann das Vlies oder der Vliesstoff Fasern mit einer längenbezogenen Masse von 1 dtex bis 150 dtex, insbesondere 1 dtex bis 10 dtex, vorzugsweise 1 dtex bis 3 dtex, aufweisen.

Ferner kann das Vlies oder der Vliesstoff eine Dicke von 0,01 mm bis 5 mm, insbesondere 0,05 mm bis 1 mm, vorzugsweise 0,1 mm bis 0,5 mm, aufweisen.

Ferner kann das Vlies oder der Vliesstoff ein Flächengewicht von 0,1 g/mm² bis 500 g/mm², insbesondere 1 g/mm² bis 100 g/mm², vorzugsweise 10 g/mm² bis 50 g/mm², aufweisen.

Ferner kann das Vlies oder der Vliesstoff eine Luftdurchlässigkeit von 10 l/m2s bis 10000 l/m2, insbesondere 100 l/m2s bis 5000 l/m2s, vorzugsweise 1000 l/m2s bis 5000 l/m2s, aufweisen.

In weiterer Ausgestaltung der Erfindung ist das textile Flächengebilde als Gewebe gestaltet.

Unter dem Ausdruck "Gewebe" soll im Sinne der vorliegenden Erfindung ein textiles Flächengebilde aus zwei Fadensystemen, nämlich Kette (Kettfäden) und Schuss (Schussfäden), verstanden werden, welche sich in der Sicht auf die Gewebefläche unter einem Winkel von genau oder annähernd 90° mustermäßig kreuzen.

Das Gewebe kann rechteck- oder quadratförmige Maschen aufweisen.

Ferner kann das Gewebe Fäden, insbesondere Garne und/oder Zwirne, und/oder Fasern mit einem Durchmesser von 1 µm bis 1 mm, insbesondere 10 µm bis 100 µm, vorzugsweise 20 µm bis 50 µm, aufweisen.

Unter dem Ausdruck "Zwirne" soll im Sinne der vorliegenden Erfindung ein linienförmiges Textil aus mehreren zusammengedrehten Garnen verstanden werden.

Ferner kann das Gewebe Fäden, insbesondere Garne und/oder Zwirne, und/oder Fasern mit einer längenbezogenen Masse von 1 dtex bis 150 dtex, insbesondere 1 dtex bis 10 dtex, vorzugsweise 1 dtex bis 3 dtex, aufweisen.

Ferner kann das Gewebe eine Dicke von 0,01 mm bis 5 mm, insbesondere 0,05 mm bis 1 mm, vorzugsweise 0,1 mm bis 0,5 mm, aufweisen.

Ferner kann das Gewebe ein Flächengewicht von 0,1 g/mm² bis 500 g/mm², insbesondere 1 g/mm² bis 100 g/mm², vorzugsweise 10 g/mm² bis 50 g/mm², aufweisen.

Ferner kann das Gewebe eine Gewebefeinheit von 0,1 Mesh bis 1000 Mesh, insbesondere 1 Mesh bis 200 Mesh, vorzugsweise 1 Mesh bis 50 Mesh, aufweisen.

Unter dem Ausdruck "Gewebefeinheit" soll im Sinne der vorliegenden Erfindung die Anzahl an Maschen des Gewebes pro englischen Zoll (25,4 mm) verstanden werden.

Ferner kann das Gewebe eine Teilung von 0,01 mm bis 100 mm, insbesondere 1 mm bis 20 mm, vorzugsweise 2 mm bis 10 mm, aufweisen.

Unter dem Ausdruck "Teilung" soll im Sinne der vorliegenden Erfindung der Abstand der Fäden und/oder Fasern des Gewebes von Fadenmitte zu Fadenmitte und/oder von Fasermitte zu Fasermitte verstanden werden. Handelt es sich bei dem Gewebe beispielsweise um ein Gewebe mit quadratförmigen Maschen, ist die Teilung in Kett- und Schussrichtung gleich.

Ferner kann das Gewebe als Leinwand-, Köper- oder Atlasgewebe gestaltet sein, d.h. die Kett- und Schussfäden können in Leinwand-, Köper- oder Atlasbindung verkreuzt sein.

Ferner kann das Gewebe eine Luftdurchlässigkeit von 10 l/m2s bis 10000 l/m2s, insbesondere 100 l/m2s bis 5000 l/m2s, vorzugsweise 1000 l/m2s bis 5000 l/m2s, aufweisen.

Alternativ kann das textile Flächengebilde als Gewirk oder Gestrick gestaltet sein.

Insbesondere kann das textile Flächengebilde netzförmig gestaltet sein.

Das textile Flächengebilde, insbesondere Fasern und/oder Fäden, insbesondere Garne und/oder Zwirne, des textilen Flächengebildes, weist ein schweißbares Material auf oder besteht aus einem solchen Material. Bei dem schweißbaren Material handelt es sich vorzugsweise um ein schweißbares Polymer. Bezüglich geeigneter schweißbarer Polymere wird auf die nachfolgend beschriebenen Polymere Bezug genommen. Dadurch ist mit besonderem Vorteil ein sicherer Verschluss oder eine sichere Fixierung des textilen Flächengebildes (unter Ausbildung einer textilen Wickelhülle oder eines textilen Wickelgebildes), insbesondere ohne zusätzliche Klebemittel, wie beispielsweise Klebestreifen, erzielbar. Erfindungsgemäß ist es daher bevorzugt, wenn das um die Hohlfasern gewickelte textile Flächengebilde ohne zusätzliche Klebemittel, insbesondere ohne Klebestreifen, fixiert oder verschlossen ist.

In weiterer Ausgestaltung der Erfindung weist das textile Flächengebilde Fasern mit einem Faserkern und einem den Faserkern vollständig umgebenden Fasermantel, d.h. Fasern mit einem Kern-Mantel-Aufbau, auf. Bevorzugt handelt es sich bei den Fasern um Mehrkomponentenfasern, insbesondere Bikomponentenfasern.

Unter dem Ausdruck "Mehrkomponentenfasern" sollen im Sinne der vorliegenden Erfindung Fasern verstanden werden, welche wenigstens zwei fest, aber trennbar miteinander verbundene Polymere mit unterschiedlichem chemischen und/oder physikalischen Aufbau aufweisen oder aus mindestens zwei derartigen Polymeren bestehen.

Unter dem Ausdruck "Bikomponentenfasern" sollen im Sinne der vorliegenden Erfindung Fasern verstanden werden, welche nur zwei fest, aber trennbar miteinander verbundene Polymere mit unterschiedlichem chemischen und/oder physikalischen Aufbau aufweisen oder nur aus zwei derartigen Polymeren bestehen.

In weiterer Ausgestaltung der Erfindung weist der Faserkern ein Faserkernpolymer und der Fasermantel ein Fasermantelpolymer auf, wobei das Faserkernpolymer einen höheren Schmelzpunkt besitzt als das Fasermantelpolymer. Dadurch ist mit besonderem Vorteil eine sichere Fixierung oder ein sicherer Verschluss des um die Hohlfasern gewickelten textilen Flächengebildes möglich, insbesondere ohne dass beispielsweise die Gefahr eines Durchschweißens des gewickelten textilen Flächengebildes und mithin einer Verletzung der umwickelten Hohlfasern besteht. So kann zur Fixierung bzw. zum Verschluss des gewickelten textilen Flächengebildes beispielsweise eine Schweißtemperatur gewählt werden, bei welcher zwar das Fasermantelpolymer, nicht jedoch das Faserkernpolymer schmilzt. Auf diese Weise kann das Durchfahren eines Schweißbalkens auf die Hohlfasern verhindert werden.

Der Schmelzpunkt des Faserkernpolymers ist wenigstens 30°C höher als der Schmelzpunkt des Fasermantelpolymers.

In weiterer Ausgestaltung der Erfindung ist das Faserkernpolymer ausgewählt aus der Gruppe bestehend aus Polyterepththalate, Polyalkylenterephthalate, Polyethylenterephthalat, Polypropylenterephthalat, Polybutylenterephthalat, Polyolefine, Polyethylen, Polyethylen hoher Dichte, Polyethylen geringer Dichte, lineares Polyethylen niederer Dichte, hochmolekulares Polyethylen, ultrahochmolekulares Polyethylen, Polypropylen und Kombinationen, insbesondere Mischungen, von wenigstens zwei der vorgenannten Faserkernpolymere.

Vorzugsweise ist das Faserkernpolymer ein Polyalkylenterephthalat, insbesondere Polyethylenterephthalat, Polypropylenterephthalat oder Polybutylenterephthalat. Besonders bevorzugt handelt es sich bei dem Faserkernpolymer um Polyethylenterephthalat.

In weiterer Ausgestaltung der Erfindung ist das Fasermantelpolymer ausgewählt aus der Gruppe bestehend aus Polyamide, Copolyamide, Polyamid 6 (PA 6 bzw. Polycaprolactam), Polyamid 6.6 (PA 6.6, d.h. Polyamid hergestellt durch Polymerisation von Hexamethylendiamin und Adipinsäure), Polyamid 6T (PA 6T, d.h. Polyamid, hergestellt durch Polymerisation von Hexamethylendiamin und Terephthalsäure), Polyamid 6.9 (PA 6.9, d.h. Polyamid, hergestellt durch Polymerisation von Hexamethylendiamin und Azelainsäure), Polyamid 6.12 (PA 6.12, d.h. Polyamid, hergestellt durch Polymerisation von Hexamethylendiamin und Dodecandisäure oder von Caprolactam und Laurinlactam), Polyamid 11 (PA 11 bzw. 11-Aminoundecansäure), Polyamid 12 (PA 12 bzw. Laurinlactam oder Omega-Aminododecansäure), Polyamid 4.6 (PA 4.6, d.h. Polyamid hergestellt durch Polymerisation von Tetramethylendiamin und Adipinsäure), Polyamid 12.12 (PA 12.12, d.h. Polyamid, hergestellt durch Polymerisation von Dodecandiamin und Dodecandisäure), Polyamid 10.10 (PA 10.10, d.h. Polyamid, hergestellt durch Polymerisation von 1,10-Decamethylendiamin und 1,10-Decandisäure) und Kombinationen, insbesondere Mischungen, von wenigstens zwei der vorgenannten Fasermantelpolymere. Vorzugsweise ist das Fasermantelpolymer Polyamid 6.

In weiterer Ausgestaltung der Erfindung ist das Faserkernpolymer ein Polyalkylenterephthalat, insbesondere Polyethylenterephthalat, und/oder das Fasermantelpolymer ein Polyamid, insbesondere Polyamid 6. Besonders bevorzugt ist das Faserkernpolymer ein Polyalkylenterephthalat, insbesondere Polyethylenterephthalat, und das Fasermantelpolymer ein Polyamid, insbesondere Polyamid 6. Durch die in diesem Absatz offenbarten Polymerkombinationen ist mit besonderem Vorteil eine besonders sichere Fixierung bzw. ein besonders sicherer Verschluss des textilen Flächengebildes, beispielsweise durch Verschweißen, erzielbar.

Bevorzugt handelt es sich bei dem textilen Flächengebilde um einen unter der Bezeichnung "Colback^{®} WA 30" kommerziell erhältlichen Vliesstoff. Hierbei handelt es sich um einen Vliesstoff mit Fasern, welche einen Kern-Mantel-Aufbau aufweisen. Der Faserkern weist Polyethylenterephthalat auf oder besteht aus Polyethylenterephthalat. Der Fasermantel weist Polyamid 6 auf oder besteht aus Polyamid 6. Ferner weist dieser Vliesstoff ein Flächengewicht von 30 g/m², eine Dicke von 0,25 mm, eine Luftdurchlässigkeit (1 mbar) von 4500 l/m²s, eine Bruchfestigkeit von 55 N/5cm, eine Bruchdehnung von 16%, eine Reißfestigkeit von 40 N, eine Steifheit von 0,3 mN sowie einen Faserdurchmesser von 37 µm auf.

Alternativ kann es sich bei dem textilen Flächengebilde bevorzugt um einen unter der Bezeichnung "Colback^{®} WA 50" kommerziell erhältlichen Vliesstoff handeln. Hierbei handelt es sich um einen Vliesstoff mit Fasern, welche einen Kern-Mantel-Aufbau aufweisen. Der Faserkern weist Polyethylenterephthalat auf oder besteht aus Polyethylenterephthalat. Der Fasermantel weist Polyamid 6 auf oder besteht aus Polyamid 6. Ferner weist dieser Vliesstoff ein Flächengewicht von 50 g/m², eine Dicke von 0,35 mm, eine Luftdurchlässigkeit (1 mbar) von 4000 l/m²s, eine Bruchfestigkeit von 150 N/5cm, eine Bruchdehnung von 22%, eine Reißfestigkeit von 75 N, eine Steifheit von 1,2 mN sowie einen Faserdurchmesser von 37 µm auf.

Alternativ kann es sich bei dem textilen Flächengebilde bevorzugt um einen unter der Bezeichnung "Colback^{®} WA 75" kommerziell erhältlichen Vliesstoff handeln. Hierbei handelt es sich um einen Vliesstoff mit Fasern, welche einen Kern-Mantel-Aufbau aufweisen. Der Faserkern weist Polyethylenterephthalat auf oder besteht aus Polyethylenterephthalat. Der Fasermantel weist Polyamid 6 auf oder besteht aus Polyamid 6. Ferner weist dieser Vliesstoff ein Flächengewicht von 75 g/m², eine Dicke von 0,5 mm, eine Luftdurchlässigkeit (1 mbar) von 3700 l/m²s, eine Bruchfestigkeit von 240 N/5cm, eine Bruchdehnung von 26%, eine Reißfestigkeit von 125 N, eine Steifheit von 3,2 mN sowie einen Faserdurchmesser von 37 µm auf.

Alternativ kann es sich bei dem textilen Flächengebilde bevorzugt um einen unter der Bezeichnung "Colback^{®} WA 100" kommerziell erhältlichen Vliesstoff handeln. Hierbei handelt es sich um einen Vliesstoff mit Fasern, welche einen Kern-Mantel-Aufbau aufweisen. Der Faserkern weist Polyethylenterephthalat auf oder besteht aus Polyethylenterephthalat. Der Fasermantel weist Polyamid 6 auf oder besteht aus Polyamid 6. Ferner weist dieser Vliesstoff ein Flächengewicht von 100 g/m², eine Dicke von 0,55 mm, eine Luftdurchlässigkeit (1 mbar) von 3400 l/m²s, eine Bruchfestigkeit von 340 N/5cm, eine Bruchdehnung von 29%, eine Reißfestigkeit von 170 N, eine Steifheit von 4,6 mN sowie einen Faserdurchmesser von 37 µm auf.

Alternativ kann es sich bei dem textilen Flächengebilde bevorzugt um einen unter der Bezeichnung "Colback^{®} WA 120" kommerziell erhältlichen Vliesstoff handeln. Hierbei handelt es sich um einen Vliesstoff mit Fasern, welche einen Kern-Mantel-Aufbau aufweisen. Der Faserkern weist Polyethylenterephthalat auf oder besteht aus Polyethylenterephthalat. Der Fasermantel weist Polyamid 6 auf oder besteht aus Polyamid 6. Ferner weist dieser Vliesstoff ein Flächengewicht von 120 g/m², eine Dicke von 0,65 mm, eine Luftdurchlässigkeit (1 mbar) von 2900 l/m²s, eine Bruchfestigkeit von 430 N/5cm, eine Bruchdehnung von 29%, eine Reißfestigkeit von 205 N, eine Steifheit von 8,3 mN sowie einen Faserdurchmesser von 37 µm auf.

In weiterer Ausgestaltung der Erfindung ist textile Flächengebilde, insbesondere das Fasermantelpolymer, durch DNA-bindende Gruppen und/oder enderotoxinbindende Gruppen und/oder mikrobenbindende Gruppen, bevorzugt durch organische Ammoniumgruppen, besonders bevorzugt quartäre Ammoniumgruppen, insbesondere vom Amin-Typ oder Imin-Typ, funktionalisiert. Entsprechend funktionalisierte textile Flächengebilde oder Fasermantelpolymere können beispielsweise durch Umsetzung eines unfunktionalisierten textilen Flächengebildes oder Fasermantelpolymers mit einer aminoreaktiven, polymerisationsfähigen Verbindung sowie durch anschließende Polymerisation mit Monomeren, welche kationische Gruppen enthalten, hergestellt werden. Alternativ können entsprechend funktionalisierte Fasermantelpolymere durch Einführung von kationischen Gruppen in das Polymerrückgrat und/oder in Seitenketten eines unfunktionalisierten Fasermantelpolymers hergestellt werden. Die Einführung der kationischen Gruppen kann dabei beispielsweise in polymeranaloger Reaktion erfolgen. Die DNA-bindenden Gruppen und/oder enderotoxinbindenden Gruppen und/oder mikrobenbindenden Gruppen verleihen dem textilen Flächengebilde in vorteilhafter Weise eine zusätzliche Funktion, nämlich die Bindung von DNA und/oder Enderotoxinen und/oder Mikroben, wie beispielsweise Bakterien und/oder Viren. Dadurch ist mit besonderem Vorteil die Reinigung eines Dialysats während des bestimmungsgemäßen Gebrauchs des Dialysators möglich.

In weiterer Ausgestaltung der Erfindung weist das textile Flächengebilde ein wenigstens teilweise wasserlösliches Polymer auf oder besteht aus einem wenigstens teilweise wasserlöslichen Polymer. Das wenigstens teilweise wasserlösliche Polymer kann dabei insbesondere nur teilweise wasserlöslich oder vollständig wasserlöslich sein. Bevorzugt handelt es sich bei dem wenigstens teilweise wasserlöslichen Polymer um ein vollständig wasserlösliches, d.h. in Wasser vollständig lösliches, Polymer. Das wenigstens teilweise wasserlösliche Polymer ist vorzugsweise ausgewählt aus der Gruppe bestehend aus Polyvinylalkohol, Polyethylenglykol, Gelatine, Stärke, Amylose, Amylopektin, Dextran, Methylcellulose, Hydroxymethylcellulose, Hydroxyhethylcellulose, Hydroxypropylcellulose, Hydroxybutylcellulose, Hydroxypropylmethylcellulose, Carboxymethylcellulose, Hyaluronsäure, Chondroitin-4-Sulfat, Chondroitin-6-Sulfat, Keratansulfat, Alginat, Alginsäure, Heparin, Heparansulfat, Chitin, Chitosan, Salze der vorgenannten wenigstens teilweise wasserlöslichen Polymere, Derivate der vorgenannten wenigstens teilweise wasserlöslichen Polymere und Kombinationen, insbesondere Mischungen, von wenigstens zwei der vorgenannten wenigstens teilweise wasserlöslichen Polymere.

Bevorzugt handelt es sich bei dem wenigstens teilweise wasserlöslichen Polymer um Polyvinylalkohol. Der Polyvinylalkohol kann ein Molekulargewicht von 5000 g/mol bis 200000 g/mol, insbesondere 10000 g/mol bis 150000 g/mol, bevorzugt 10000 g/mol bis 100000 g/mol, aufweisen.

Ferner kann das textile Flächengebilde ein wasserunlösliches Polymer aufweisen oder aus einem wasserunlöslichen Polymer bestehen. Das wasserunlösliche Polymer ist vorzugsweise ausgewählt aus der Gruppe bestehend aus Polyolefine, Polyethylen, Polyethylen mit niedriger Dichte, Polyethylen mit hoher Dichte, hochmolekulares Polyethylen, ultrahochmolekulares Polyethylen, Polypropylen, Polyethylenterephthalat, Polypropylenterephthalat, Polybutylenterephthalat, Polyacrylnitril, PA 6, PA 6.6, PA 6.12, PA 12, Seide, Polytetrafluorethylen, Polyvinylidendifluorid, Polytetrafluorpropylen, Polyhexafluorpropylen, Polyhydroxyalkansäuren bzw. Polyhydroxyalkanoate, Polyglykolsäure bzw. Polyglykolid, Polymilchsäure bzw. Polylactid, Polydioxanon, Poly-3-Hydroxybuttersäure bzw. Poly-3-Hydroxybutyrat, Poly-4-Hydroxybuttersäure bzw. Poly-4-Hydroxybutyrat, Polytrimethylencarbonat, Poly-ε-Caprolacton, Salze der vorgenannten wasserunlöslichen Polymere, Derivate der vorgenannten wasserunlöslichen Polymere, Copolymere der vorgenannten wasserunlöslichen Polymere und Kombinationen, insbesondere Mischungen, von wenigstens zwei der vorgenannten wasserunlöslichen Polymere.

Ferner ist es bevorzugt, wenn das umwickelte Hohlfaserbündel außerhalb des Gehäuses des Dialysators ein Übermaß von 1% bis 30% aufweist, bezogen auf den Innendurchmesser des Dialysatorgehäuses. Das Übermaß errechnet sich dabei aus der nachfolgenden Formel I: $Db = Dgi ⋅ \left(1+X\right) ,$ wobei Db der Durchmesser des umwickelten Hohlfaserbündels, Dgi der Innendurchmesser des Gehäuses des Dialysators und X der Übermaßfaktor sind, wobei X einen Wert von 0,01 bis 0,3 annehmen kann.

Ferner ist es bevorzugt, wenn das gewickelte textile Flächengebilde spaltfrei an der Innenseite der Wandung des Dialysatorgehäuses anliegt.

In weiterer Ausgestaltung der Erfindung umwickelt das textile Flächengebilde die Hohlfasern unter Ausbildung eines wenigstens abschnittsweise, insbesondere nur abschnittsweise, dreilagigen Wickelaufbaus. Vorzugsweise sind nur eine äußere Lage und eine mittlere Lage des dreilagigen Wickelaufbaus miteinander verbunden, insbesondere durch eine stoffschlüssige Verbindung, vorzugsweise durch eine Schweißverbindung. Die stoffschlüssige Verbindung, vorzugsweise Schweißverbindung, erstreckt sich bevorzugt in Längsrichtung des gewickelten textilen Flächengebildes. Ferner ist es bevorzugt, dass die mittlere Lage und eine innere Lage des dreilagigen Wickelaufbaus nicht miteinander verbunden sind, insbesondere nicht durch eine stoffschlüssige Verbindung wie Schweißverbindung. Dadurch kann das Risiko einer Beschädigung der durch das textile Flächengebilde umwickelten Hohlfasern signifikant reduziert oder sogar vollständig eliminiert werden. Die innere Lage des dreilagigen Wickelaufbaus dient somit vorzugsweise ausschließlich als Schutzschicht für die umwickelten Hohlfasern. Unter dem Ausdruck "äußere Lage" soll hierbei im Sinne der vorliegenden Erfindung die Lage des dreilagigen Wickelaufbaus verstanden werden, welche im Verhältnis zur mittleren Lage und inneren Lage des dreilagigen Wickelaufbaus den größten Abstand zu den umwickelten Hohlfasern aufweist. Unter dem Ausdruck "innere Lage" soll im Sinne der vorliegenden Erfindung die Lage des dreilagigen Wickelaufbaus verstanden werden, welche im Verhältnis zur äußeren Lage und mittleren Lage des dreilagigen Wickelaufbaus den geringsten Abstand zu den umwickelten Hohlfasern aufweist. Unter dem Ausdruck "mittlere Lage" soll im Sinne der vorliegenden Erfindung die Lage des dreilagigen Wickelaufbaus verstanden werden, welche zwischen der äußeren Lage und der inneren Lage des dreilagigen Wickelaufbaus angeordnet ist.

Gemäß einem zweiten Aspekt betrifft die Erfindung ein Dialysegerät, welches einen Dialysator gemäß erstem Erfindungsaspekt aufweist.

Bezüglich weiterer Merkmale und Vorteile des Dialysators wird vollständig auf die im Rahmen des ersten Erfindungsaspekts gemachten Ausführungen sowie auf die noch folgende Beschreibung Bezug genommen. Die dort in Bezug auf den Dialysator beschriebenen Merkmale und Vorteile gelten sinngemäß auch in Bezug auf das Dialysegerät gemäß zweitem Erfindungsaspekt.

Gemäß einem dritten Aspekt betrifft die Erfindung ein Kit, insbesondere zum Herstellen eines Dialysators gemäß erstem Erfindungsaspekt.

Das Kit weist Folgendes auf:
- eine mit einem textilen Flächengebilde umwickelte Vielzahl von Hohlfasern, wobei das textile Flächengebilde flüssigkeitsdurchlässig ist. Insbesondere kann das textile Flächengebilde wasser- und/oder dialysatdurchlässig, und/oder wenigstens teilweise, insbesondere nur teilweise oder vollständig, wasserlöslich gestaltet sein, wobei das textile Flächengebilde Fasern mit einem Faserkern und einem den Faserkern vollständig umgebenden Fasermantel aufweist, wobei der Faserkern ein Faserkernpolymer und der Fasermantel ein Fasermantelpolymer aufweist, wobei das Faserkernpolymer einen höheren Schmelzpunkt besitzt als das Fasermantelpolymer, wobei der Schmelzpunkt des Faserkernpolymers um wenigstens 30°C höher ist als der Schmelzpunkt des Fasermantelpolymer, und
- eine Einführhilfe zum Einführen der mit dem textilen Flächengebilde umwickelten Vielzahl von Hohlfasern in ein Gehäuse des Dialysators.

Bei der Einführhilfe handelt es sich vorzugsweise um eine trichterförmige Einführhilfe.

Bezüglich weiterer Merkmale und Vorteile des Kits wird vollständig auf die im Rahmen des ersten Erfindungsaspekts gemachten Ausführungen sowie auf die noch folgende Beschreibung Bezug genommen. Die dort in Bezug auf den Dialysator, insbesondere die Vielzahl von Hohlfasern, das Gehäuse des Dialysators sowie das textile Flächengebilde, beschriebenen Merkmale und Vorteile gelten sinngemäß auch in Bezug auf das Kit gemäß drittem Erfindungsaspekt.

Gemäß einem vierten Aspekt betrifft die Erfindung ein Verfahren zum Herstellen eines Dialysators, insbesondere gemäß erstem Erfindungsaspekt.

Das Verfahren weist die nachfolgenden Schritte auf:
- Umwickeln einer Vielzahl von Hohlfasern mit einem textilen Flächengebilde, wobei das textile Flächengebilde flüssigkeitsdurchlässig ist. Insbesondere kann das textile Flächengebilde wasser- und/oder dialysatdurchlässig, und/oder wenigstens teilweise, insbesondere nur teilweise oder vollständig, wasserlöslich gestaltet sein, wobei das textile Flächengebilde Fasern mit einem Faserkern und einem den Faserkern vollständig umgebenden Fasermantel aufweist, wobei der Faserkern ein Faserkernpolymer und der Fasermantel ein Fasermantelpolymer aufweist, wobei das Faserkernpolymer einen höheren Schmelzpunkt besitzt als das Fasermantelpolymer, wobei der Schmelzpunkt des Faserkernpolymers um wenigstens 30°C höher ist als der Schmelzpunkt des Fasermantelpolymers, und
- Einführen der mit dem textilen Flächengebilde umwickelten Vielzahl von Hohlfasern in ein Gehäuse des Dialysators.

Die Hohlfasern werden bevorzugt mittels einer Hohlfaserspinnanlage, insbesondere mittels einer kontinuierlich arbeitenden Hohlfaserspinnanlage, hergestellt.

Vorzugsweise werden die aus der Hohlfaserspinnanlage austretenden Hohlfasern, in der Regel chargenweise, aufgewickelt. Die Hohlfasern können mittels einer Haspelvorrichtung, beispielsweise mittels eines Haspelrads, aufgewickelt werden. Die Haspelvorrichtung weist bevorzugt gleichmäßig auf Umfang verteilte Wickeldorne auf, auf welchen die Hohlfasern aufliegen können. Dadurch können die Hohlfasern zwischen den Wickeldornen frei gespannt werden. Die Abstände zwischen den Wickeldornen können vorzugsweise so gewählt werden, dass zwischen jeweils zwei Wickeldornen ein Hohlfaserbündel entsteht. Diese Hohlfaserbündel werden mittels des textilen Flächengebildes vorzugsweise so umwickelt, dass das Hohlfaserbündel einen kreisförmigen Querschnitt mit vorher festgelegtem definiertem Durchmesser erhält.

Bevorzugt werden so viele Hohlfasern von dem textilen Flächengebilde umwickelt, dass das hieraus resultierende umwickelte Hohlfaserbündel ein Übermaß von 1% bis 30% aufweist, bezogen auf den Innendurchmesser des Gehäuses des Dialysators.

Ferner ist es bevorzugt, dass die die Hohlfasern mit dem textilen Flächengebilde unter Ausbildung eines wenigstens abschnittsweise, insbesondere nur abschnittsweise, dreilagigen Wickelaufbaus umwickelt werden. Vorzugsweise werden nur eine äußere Lage und eine mittlere Lage des dreilagigen Wickelaufbaus miteinander verbunden, insbesondere durch eine stoffschlüssige Verbindung, vorzugsweise durch eine Schweißverbindung. Insbesondere werden die äußere Lage und die mittlere Lage des dreilagigen Wickelaufbaus in Längsrichtung des gewickelten textilen Flächengebildes stoffschlüssig miteinander verbunden, bevorzugt miteinander verschweißt. Ferner ist es bevorzugt, dass die mittlere Lage und eine innere Lage des dreilagigen Wickelaufbaus nicht miteinander verbunden sind, insbesondere nicht durch eine stoffschlüssige Verbindung wie Schweißverbindung.

Besonders bevorzugt werden die äußere Lage und die mittlere Lage des dreilagigen Wickelaufbaus durch Wärmeimpulsschweißen miteinander verbunden. Alternativ können die äußere Lage und die mittlere Lage des dreilagigen Wickelaufbaus durch andere Kunststoffschweißverfahren, wie beispielsweise beheizbare Werkzeuge oder Laserschweißverfahren, miteinander verbunden werden.

Bezüglich weiterer Merkmale und Vorteile des Verfahrens wird vollständig auf die im Rahmen des ersten Erfindungsaspekts gemachten Ausführungen sowie auf die noch folgende Beschreibung Bezug genommen. Die dort in Bezug auf den Dialysator, insbesondere die Vielzahl von Hohlfasern, das Gehäuse des Dialysators sowie das textile Flächengebilde, beschriebenen Merkmale und Vorteile gelten sinngemäß auch in Bezug auf das Verfahren gemäß viertem Erfindungsaspekt.

Weitere Merkmale und Vorteile der Erfindung ergeben sich aus den Ansprüchen sowie aus der nachfolgenden Beschreibung bevorzugter Ausführungsbeispiele der Erfindung, welche anhand der Zeichnungen dargestellt sind.

### FIGURENKURZBESCHREIBUNGEN

- Fig. 1: zeigt eine schematische Längsschnittdarstellung einer Ausführungsform eines erfindungsgemäßen Dialysators,
- Fig. 2: zeigt schematisch eine vergrößerte Detaildarstellung eines gewickelten textilen Flächengebildes eines erfindungsgemäßen Dialysators in einem Bereich A nach Fig. 1,
- Fig. 3: zeigt schematisch eine vergrößerte Detaildarstellung eines alternativen gewickelten textilen Flächengebildes eines erfindungsgemäßen Dialysators in einem Bereich A nach Fig. 1,
- Fig. 4: zeigt eine schematische Querschnittsdarstellung eines erfindungsgemäßen Dialysators entlang einer Schnittlinie IV-IV nach Fig.1 und
- Fig. 5: zeigt schematisch eine Ausführungsform eines erfindungsgemäßen Kits.

### AUSFÜHRLICHE FIGURENBESCHREIBUNGEN

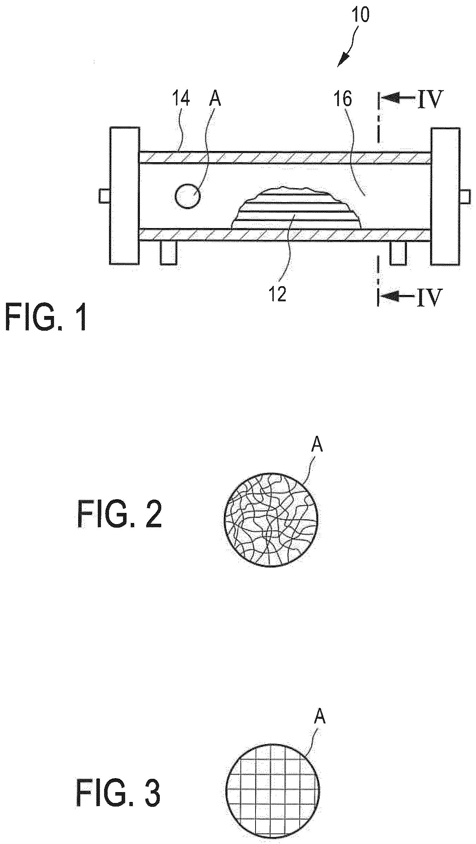

Fig. 1 zeigt schematisch eine Längsschnittdarstellung einer Ausführungsform eines Dialysators 10 gemäß der vorliegenden Erfindung.

Der Dialysator 10 weist eine Vielzahl von Hohlfasern 12 auf. Die Hohlfasern 12 sind in einem Gehäuse 14 des Dialysators 10 angeordnet. Dabei sind die Hohlfasern 12 in Längsrichtung des Gehäuses 14 erstreckt und quer zur Längsrichtung des Gehäuses 14 nebeneinander angeordnet. Bevorzugt sind die Hohlfasern 12 parallel oder im Wesentlichen parallel zueinander angeordnet. Axiale Endabschnitte der Hohlfasern 12 sind vorzugsweise in einer (nicht dargestellten) Hohlfasereinbettung, beispielsweise aus Polyurethan, aufgenommen.

Das Gehäuse 14 ist vorzugsweise röhrenförmig, insbesondere rundröhrenförmig, gestaltet. Das Gehäuse 14 kann ferner aus einem Kunststoff hergestellt sein.

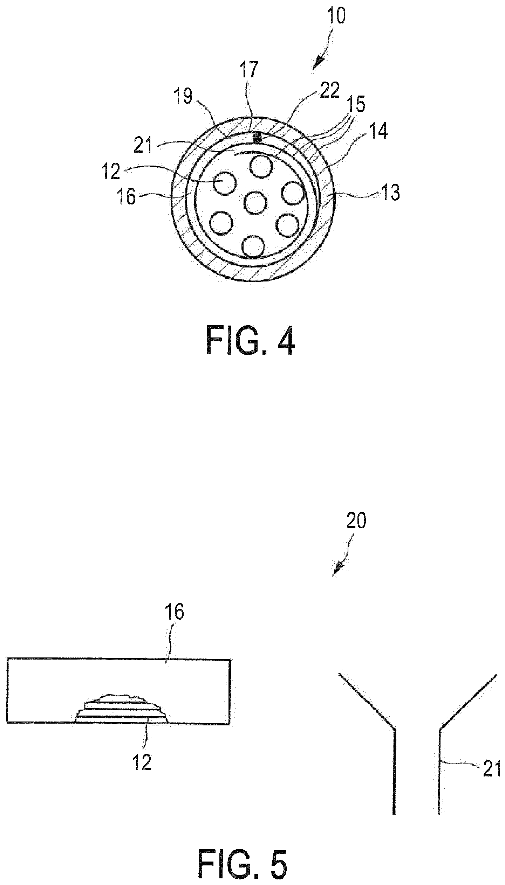

Die Hohlfasern 12 sind in dem Gehäuse 14 mit einem textilen Flächengebilde 16 umwickelt. Das um die Hohlfasern 12 gewickelte textile Flächengebilde 16 bildet somit eine textile Wickelhülle oder textiles Wickelgebilde. Das gewickelte textile Flächengebilde 16 ist vorzugsweise durch eine sich in Längsrichtung des gewickelten textilen Flächengebildes 16 erstreckende stoffschlüssige Verbindung, vorzugsweise Schweißverbindung, 22 (siehe Fig. 4) fixiert.

Durch das textile Flächengebilde 16 sind die Hohlfasern 12 vorzugsweise zu einem Hohlfaserbündel, insbesondere mit kreisförmigem Querschnitt, zusammengefasst.

Das textile Flächengebilde 16 ist flüssigkeitsdurchlässig, insbesondere wasser- und/oder dialysatdurchlässig, und/oder wenigstens teilweise, bevorzugt vollständig, wasserlöslich gestaltet. Dadurch kann das textile Flächengebilde 16 in dem Dialysatorgehäuse 14 verbleiben. Mithin ist ein Entfernen des textilen Flächengebildes 16 aus dem Dialysatorgehäuse 14 entbehrlich, wodurch die im Zusammenhang von gattungsgemäßen Dialysatoren bekannten, knickfaserbedingten Nachteile vermieden werden können.

Bevorzugt besitzt das textile Flächengebilde 16 einen Porendurchmesser, insbesondere mittleren Porendurchmesser, von 0,1 µm bis 10 mm, insbesondere 200 µm bis 1 mm, vorzugsweise 1 mm bis 5 mm, auf. Insbesondere durch die vorgenannten Porendurchmesser kann mit besonderem Vorteil gewährleistet werden, dass das textile Flächengebilde 16 weder beim Vergießen der Hohlfasern 12 mit einer Vergussmasse zur Herstellung einer Hohlfasereinbettung noch einem Dialysatfluss während eines bestimmungsgemäßen Gebrauchs des Dialysators 10 einen nennenswerten Widerstand bietet.

Ferner kann es bevorzugt sein, dass das textile Flächengebilde 16 Fasern mit einem Kern-Mantel-Aufbau, d.h. Fasern mit einem Faserkern und einem den Faserkern wenigstens teilweise, vorzugsweise vollständig, umgebenden Fasermantel aufweist. Vorzugsweise weist der Faserkern ein Faserkernpolymer und der Fasermantel ein Fasermantelpolymer auf, wobei das Faserkernpolymer bevorzugt einen höheren Schmelzpunkt aufweist als das Fasermantelpolymer. Bei dem Faserkernpolymer kann es sich insbesondere um ein Polyalkylenterephthalat, vorzugsweise Polyethylenterephthalat, und bei dem Fasermantelpolymer um ein Polyamid, bevorzugt Polyamid 6, handeln. Dadurch kann beispielsweise im Falle einer Schweißfixierung des gewickelten textilen Flächengebildes 16 bei Anwendung einer Schweißtemperatur, bei welcher zwar das Fasermantelpolymer, nicht jedoch das Faserkernpolymer schmilzt, ein Durchschweißen des gewickelten textilen Flächengebildes 16 und mithin eine Beschädigung der Hohlfasern 12 vermieden werden.

Alternativ kann das textile Flächengebilde 16 wenigsten teilweise, insbesondere vollständig, aus einem wasserlöslichen Polymer, bevorzugt Polyvinylalkohol, gefertigt sein.

Fig. 2 zeigt schematisch eine vergrößerte Detaildarstellung eines gewickelten textilen Flächengebildes 16 eines erfindungsgemäßen Dialysators 10 in einem Bereich A nach Fig. 1. Das textile Flächengebilde 16 ist als Vliesstoff gestaltet. Der Vliesstoff kann beispielsweise Fasern mit einem Kern-Mantel-Aufbau, wie im Zusammenhang von Fig. 1 beschrieben, und ein Flächengewicht von 30 g/m², eine Dicke von 0,25 mm, eine Luftdurchlässigkeit (1 mbar) von 4500 l/m²s, eine Bruchfestigkeit von 55 N/5cm, eine Bruchdehnung von 16%, eine Reißfestigkeit von 40 N, eine Steifheit von 0,3 mN sowie einen Faserdurchmesser von 37 µm aufweisen. Ein solcher Vliesstoff ist unter der Bezeichnung "Colback^{®} WA 30" kommerziell erhältlich.

Fig. 3 zeigt schematisch eine vergrößerte Detaildarstellung eines alternativen gewickelten textilen Flächengebildes 16 eines erfindungsgemäßen Dialysators 10 in einem Bereich A nach Fig. 1. Das textile Flächengebilde 16 ist als Gewebe gestaltet. Insbesondere die vorgenannten Maschenweiten stellen in vorteilhafter Weise einen gegenüber einer zur Herstellung einer Hohlfasereinbettung verwendeten Vergussmasse sowie gegenüber einem Dialysatfluss vernachlässigbaren Widerstand dar.

Fig. 4 zeigt schematisch eine Querschnittsdarstellung eines Dialysators 10 nach Fig. 1. Das textile Flächengebilde 16 umwickelt die Hohlfasern 12 unter Ausbildung eines wenigstens abschnittsweise, insbesondere nur abschnittsweise, dreilagigen Wickelaufbaus 15 mit einer äußeren Lage 17, einer mittleren Lage 19 sowie einer inneren Lage 21. Vorzugsweise sind nur die äußere Lage 17 und die mittlere Lage 19 durch eine stoffschlüssige Verbindung, vorzugsweise Schweißverbindung, 22 miteinander verbunden. Mit anderen Worten ist die innere Lage 21 vorzugsweise nicht in die stoffschlüssige Verbindung, insbesondere Schweißverbindung, 22 einbezogen. Die innere Lage 21 dient somit vorzugsweise ausschließlich als Schutzschicht für die Hohlfasern 12. Dadurch kann eine Beschädigung der Hohlfasern 12 beim Fixieren bzw. Verschluss des gewickelten textilen Flächengebildes 16 vermieden werden. Bevorzugt liegt das gewickelte textile Flächengebilde 16 spaltfrei an der Innenseite der Wandung 13 des Gehäuses 14 an.

Fig. 5 zeigt schematisch eine Ausführungsform eines erfindungsgemäßen Kits 20.

Das Kit 20 weist ein textiles Flächengebilde 16 auf, welches eine Vielzahl von Hohlfasern 12 umwickelt. Ferner weist das Kit 20 eine Einführhilfe 21 zum Einführen der mit dem textilen Flächengebilde 16 umwickelten Vielzahl von Hohlfasern 12 in ein Gehäuse eines Dialysators auf. Die Einführhilfe ist vorzugsweise trichterförmig gestaltet.

Bezüglich weiterer Merkmale und Vorteile des textilen Flächengebildes 16 sowie der Vielzahl von Hohlfasern 12 wird zur Vermeidung von Wiederholungen vollständig auf die Figurenbeschreibungen zu den Fig. 1 bis 4 Bezug genommen. Die dort insbesondere in Bezug auf das textile Flächengebilde 16, die Hohlfasern 12 sowie den Dialysator 10 beschriebenen Merkmale und Vorteile gelten sinngemäß auch für das Kit 20.

## Patentansprüche

1. Dialysator (10), aufweisend eine Vielzahl von Hohlfasern (12), welche in einem Gehäuse (14) des Dialysators (10) angeordnet sind, wobei die Vielzahl von Hohlfasern (12) in dem Gehäuse (14) des Dialysators (10) mit einem textilen Flächengebilde (16) umwickelt ist, wobei das textile Flächengebilde (16) flüssigkeitsdurchlässig gestaltet ist, **dadurch gekennzeichnet, dass** das textile Flächengebilde (16) Fasern mit einem Faserkern und einem den Faserkern vollständig umgebenden Fasermantel aufweist, wobei der Faserkern ein Faserkernpolymer und der Fasermantel ein Fasermantelpolymer aufweist, wobei das Faserkernpolymer einen höheren Schmelzpunkt besitzt als das Fasermantelpolymer, wobei der Schmelzpunkt des Faserkernpolymers um wenigstens 30 °C höher ist als der Schmelzpunkt des Fasermantelpolymers.

2. Dialysator (10) nach Anspruch 1, **dadurch gekennzeichnet, dass** das textile Flächengebilde (16) einen Porendurchmesser, insbesondere mittleren Porendurchmesser, von 0,1 mm bis 10 mm, insbesondere 200 µm bis 1 mm, vorzugsweise 1 mm bis 5 mm, aufweist.

3. Dialysator (10) nach Anspruch 1 oder 2, **dadurch gekennzeichnet, dass** das textile Flächengebilde (16) ein Vliesstoff ist.

4. Dialysator (10) nach Anspruch 1, **dadurch gekennzeichnet, dass** das textile Flächengebilde (16) ein Gewebe ist.

5. Dialysator (10) nach einem der vorhergehenden Ansprüche, **dadurch gekennzeichnet, dass** die Fasern Bikomponentenfasern sind.

6. Dialysator (10) nach einem der vorhergehenden Ansprüche, **dadurch gekennzeichnet, dass** das Faserkernpolymer ausgewählt ist aus der Gruppe bestehend aus Polyterepththalate, Polyalkylenterephthalate, Polyethylenterephthalat, Polypropylenterephthalat, Polybutylenterephthalat, Polyolefine, Polyethylen, Polyethylen hoher Dichte, Polyethylen geringer Dichte, lineares Polyethylen niederer Dichte, hochmolekulares Polyethylen, ultrahochmolekulares Polyethylen, Polypropylen und Kombinationen, insbesondere Mischungen, von wenigstens zwei der vorgenannten Faserkernpolymere.

7. Dialysator (10) nach einem der vorhergehenden Ansprüche, **dadurch gekennzeichnet, dass** das Fasermantelpolymer ausgewählt ist aus der Gruppe bestehend aus Polyamide, Copolyamide, Polyamid 6, Polyamid 6.6, Polyamid 6T, Polyamid 6.9, Polyamid 6.12, Polyamid 11, Polyamid 12, Polyamid 4.6, Polyamid 12.12, Polyamid 10.10 und Kombinationen, insbesondere Mischungen, von wenigstens zwei der vorgenannten Fasermantelpolymere.

8. Dialysator (10) nach einem der vorhergehenden Ansprüche, **dadurch gekennzeichnet, dass** das Faserkernpolymer ein Polyalkylenterephthalat, insbesondere Polyethylenterephthalat, und/oder das Fasermantelpolymer ein Polyamid, insbesondere Polyamid 6 ist.

9. Dialysator (10) nach einem der vorhergehenden Ansprüche, **dadurch gekennzeichnet, dass** das textile Flächengebilde, insbesondere das Fasermantelpolymer, durch DNA-bindende Gruppen und/oder endotoxinbindende Gruppen und/oder mikrobenbindende Gruppen, bevorzugt organische Ammoniumgruppen, besonders bevorzugt quartäre Ammoniumgruppen, insbesondere vom Amin-Typ oder Imin-Typ, funktionalisiert ist.

10. Dialysator (10) nach einem der vorhergehenden Ansprüche, **dadurch gekennzeichnet, dass** das textile Flächengebilde (16) die Hohlfasern (12) unter Ausbildung eines wenigstens abschnittsweise dreilagigen Wickelaufbaus umwickelt, wobei vorzugsweise nur eine äußere Lage (17) und eine mittlere Lage (19) des dreilagigen Wickelaufbaus durch eine stoffschlüssige Verbindung, vorzugsweise Schweißverbindung, (22) miteinander verbunden sind.

11. Dialysegerät, aufweisend einen Dialysator (10) nach einem der vorhergehenden Ansprüche.

12. Kit (20) zum Herstellen eines Dialysators (10) nach einem der Ansprüche 1 bis 10, aufweisend
- eine mit einem textilen Flächengebilde (16) umwickelte Vielzahl von Hohlfasern (12), wobei das textile Flächengebilde (16) flüssigkeitsdurchlässig gestaltet ist, wobei das textile Flächengebilde (16) Fasern mit einem Faserkern und einem den Faserkern vollständig umgebenden Fasermantel aufweist, wobei der Faserkern ein Faserkernpolymer und der Fasermantel ein Fasermantelpolymer aufweist, wobei das Faserkernpolymer einen höheren Schmelzpunkt besitzt als das Fasermantelpolymer, wobei der Schmelzpunkt des Faserkernpolymers um wenigstens 30 °C höher ist als der Schmelzpunkt des Fasermantelpolymers, und
- eine Einführhilfe (21) zum Einführen der mit dem textilen Flächengebilde (16) umwickelten Vielzahl von Hohlfasern (12) in ein Gehäuse (14) des Dialysators (10).

13. Verfahren zum Herstellen eines Dialysators (10) nach einem der Ansprüche 1 bis 11, aufweisend die nachfolgenden Schritte:
- Umwickeln einer Vielzahl von Hohlfasern (12) mit einem textilen Flächengebilde (16), wobei das textile Flächengebilde (16) flüssigkeitsdurchlässig gestaltet ist, wobei das textile Flächengebilde (16) Fasern mit einem Faserkern und einem den Faserkern vollständig umgebenden Fasermantel aufweist, wobei der Faserkern ein Faserkernpolymer und der Fasermantel ein Fasermantelpolymer aufweist, wobei das Faserkernpolymer einen höheren Schmelzpunkt besitzt als das Fasermantelpolymer, wobei der Schmelzpunkt des Faserkernpolymers um wenigstens 30 °C höher ist als der Schmelzpunkt des Fasermantelpolymers und
- Einführen der mit dem textilen Flächengebilde (16) umwickelten Vielzahl von Hohlfasern (12) in ein Gehäuse (14) des Dialysators (10).

## Claims

1. A dialyzer (10), comprising a multiplicity of hollow fibers (12) disposed in a housing (14) of the dialyzer (10), wherein the multiplicity of hollow fibers (12) in the housing (14) of the dialyzer (10) are wrapped with a textile fabric (16), the textile fabric (16) being fluid-permeable in design, **characterized in that** the textile fabric (16) comprises fibers having a fiber core and a fiber sheath completely surrounding the fiber core, wherein the fiber core comprises a fiber core polymer and the fiber sheath comprises a fiber sheath polymer, the melting point of the fiber core polymer being higher than that of the fiber sheath polymer, wherein the melting point of the fiber core polymer is at least 30°C higher than the melting point of the fiber sheath polymer.

2. The dialyzer (10) as claimed in claim 1, **characterized in that** the textile fabric (16) has a pore diameter, more particularly mean pore diameter, of 0.1 mm to 10 mm, more particularly 200 µm to 1 mm, preferably 1 mm to 5 mm.

3. The dialyzer (10) as claimed in claim 1 or 2, **characterized in that** the textile fabric (16) is a nonwoven fabric.

4. The dialyzer (10) as claimed in claim 1, **characterized in that** the textile fabric (16) is a woven fabric.

5. The dialyzer (10) as claimed in any of the preceding claims, **characterized in that** the fibers are bicomponent fibers.

6. The dialyzer (10) as claimed in any of the preceding claims, **characterized in that** the fiber core polymer is selected from the group consisting of polyterephthalates, polyalkylene terephthalates, polyethylene terephthalate, polypropylene terephthalate, polybutylene terephthalate, polyolefins, polyethylene, high-density polyethylene, low-density polyethylene, linear low-density polyethylene, high molecular mass polyethylene, ultra-high molecular mass polyethylene, polypropylene, and combinations, more particularly mixtures, of at least two of the aforesaid fiber core polymers.

7. The dialyzer (10) as claimed in any of the preceding claims, **characterized in that** the fiber sheath polymer is selected from the group consisting of polyamides, copolyamides, polyamide 6, polyamide 6.6, polyamide 6T, polyamide 6.9, polyamide 6.12, polyamide 11, polyamide 12, polyamide 4.6, polyamide 12.12, polyamide 10.10, and combinations, more particularly mixtures, of at least two of the aforesaid fiber sheath polymers.

8. The dialyzer (10) as claimed in any of the preceding claims, **characterized in that** the fiber core polymer is a polyalkylene terephthalate, more particularly polyethylene terephthalate, and/or the fiber sheath polymer is a polyamide, more particularly polyamide 6.

9. The dialyzer (10) as claimed in any of the preceding claims, **characterized in that** the fabric, more particularly the fiber sheath polymer, is functionalized by DNA-binding groups and/or enderotoxin-binding groups and/or microbe-binding groups, preferably organic ammonium groups, more preferably quaternary ammonium groups, more particularly of the amine type or imine type.

10. The dialyzer (10) as claimed in any of the preceding claims, **characterized in that** the textile fabric (16) wraps the hollow fibers (12) to form a wrapped construction which is three-ply at least in sections, where preferably only an outer ply (17) and a middle ply (19) of the three-ply wrapped construction are joined to one another through a materially bonded connection, preferably welded connection, (22).

11. A dialysis machine comprising a dialyzer (10) as claimed in any of the preceding claims.

12. A kit (20) for producing a dialyzer (10) as claimed in any of claims 1 to 10, comprising
- a multiplicity of hollow fibers (12) wrapped with a textile fabric (16), the textile fabric (16) being fluid-permeable in design, wherein the textile fabric (16) comprises fibers having a fiber core and a fiber sheath completely surrounding the fiber core, wherein the fiber core comprises a fiber core polymer and the fiber sheath comprises a fiber sheath polymer, the melting point of the fiber core polymer being higher than that of the fiber sheath polymer, wherein the melting point of the fiber core polymer is at least 30°C higher than the melting point of the fiber sheath polymer, and
- an insertion aid (21) for inserting the multiplicity of hollow fibers (12) wrapped with the textile fabric (16) into a housing (14) of the dialyzer (10).

13. A process for producing a dialyzer (10) as claimed in any of claims 1 to 11, comprising the following steps:
- wrapping a multiplicity of hollow fibers (12) with a textile fabric (16), the textile fabric (16) being fluid-permeable in design, wherein the textile fabric (16) comprises fibers having a fiber core and a fiber sheath completely surrounding the fiber core, wherein the fiber core comprises a fiber core polymer and the fiber sheath comprises a fiber sheath polymer, the melting point of the fiber core polymer being higher than that of the fiber sheath polymer, wherein the melting point of the fiber core polymer is at least 30°C higher than the melting point of the fiber sheath polymer, and
- inserting the multiplicity of hollow fibers (12) wrapped with the textile fabric (16) into a housing (14) of the dialyzer (10).

## Revendications

1. Dialyseur (10), présentant une pluralité de fibres creuses (12) qui sont agencées dans un boîtier (14) du dialyseur (10), dans lequel la pluralité de fibres creuses (12) dans le boîtier (14) du dialyseur (10) est enroulée dans une structure plate textile (16), la structure plate textile (16) étant réalisée de manière perméable aux liquides, **caractérisé en ce que** la structure plate textile (16) présente des fibres ayant un cœur de fibre et une enveloppe de fibre entourant entièrement le cœur de fibre, dans lequel le cœur de fibre présente un polymère de cœur de fibre et l'enveloppe de fibre présente un polymère d'enveloppe de fibre, le polymère de cœur de fibre ayant un point de fusion plus élevé que le polymère d'enveloppe de fibre, le point de fusion du polymère de cœur de fibre étant supérieur d'au moins 30 °C au point de fusion du polymère d'enveloppe de fibre.

2. Dialyseur (10) selon la revendication 1, **caractérisé en ce que** la structure plate textile (16) présente un diamètre de pore, en particulier un diamètre de pore moyen, de 0,1 mm à 10 mm, en particulier de 200 µm à 1 mm, de préférence de 1 mm à 5 mm.

3. Dialyseur (10) selon la revendication 1 ou 2, **caractérisé en ce que** la structure plate textile (16) est un non-tissé.

4. Dialyseur (10) selon la revendication 1, **caractérisé en ce que** la structure plate textile (16) est un tissu.

5. Dialyseur (10) selon l'une quelconque des revendications précédentes, **caractérisé en ce que** les fibres sont des fibres bicomposantes.

6. Dialyseur (10) selon l'une quelconque des revendications précédentes, **caractérisé en ce que** le polymère de cœur de fibre est sélectionné dans le groupe composé de polytéréphtalates, de polyalkylène téréphtalates, de polyéthylène téréphtalate, de polypropylène téréphtalate, de polybutylène téréphtalate, de polyoléfines, de polyéthylène, de polyéthylène haute densité, de polyéthylène basse densité, de polyéthylène linéaire basse densité, de polyéthylène à haute masse moléculaire, de polyéthylène à ultra-haute masse moléculaire, de polypropylène et de combinaisons, en particulier de mélanges, d'au moins deux des polymères de cœur de fibre ci-dessus.

7. Dialyseur (10) selon l'une quelconque des revendications précédentes, **caractérisé en ce que** le polymère d'enveloppe de fibre est sélectionné dans le groupe composé de polyamides, de copolyamides, de polyamide 6, de polyamide 6.6, de polyamide 6T, de polyamide 6.9, de polyamide 6.12, de polyamide 11, de polyamide 12, de polyamide 4.6, de polyamide 12.12, de polyamide 10.10 et de combinaisons, en particulier de mélanges, d'au moins deux des polymères d'enveloppe de fibre ci-dessus.

8. Dialyseur (10) selon l'une quelconque des revendications précédentes, **caractérisé en ce que** le polymère de cœur de fibre est un polyalkylène téréphtalate, en particulier du polyéthylène téréphtalate, et/ou le polymère d'enveloppe de fibre est un polyamide, en particulier du polyamide 6.

9. Dialyseur (10), selon l'une quelconque des revendications précédentes, **caractérisé en ce que** la structure plate textile, en particulier le polymère d'enveloppe de fibre, est fonctionnalisée par des groupes se liant à l'ADN et/ou des groupes se liant aux endotoxines et/ou des groupes se liant aux microbes, de préférence des groupes d'ammonium organiques, de manière particulièrement préférée des groupes d'ammonium quaternaires, en particulier de type amine ou de type imine.

10. Dialyseur (10) selon l'une quelconque des revendications précédentes, **caractérisé en ce que** la structure plate textile (16) enroule les fibres creuses (12) en réalisant un ensemble d'enroulement à trois couches au moins par endroits, dans lequel de préférence seule une couche extérieure (17) et une couche centrale (19) de l'ensemble d'enroulement à trois couches sont reliées ensemble par une liaison par liaison de matière, de préférence une liaison soudée (22).

11. Appareil de dialyse, présentant un dialyseur (10) selon l'une quelconque des revendications précédentes.

12. Kit (20) de fabrication d'un dialyseur (10) selon l'une quelconque des revendications 1 à 10, présentant
- une pluralité de fibres creuses (12) entourées d'une structure plate textile (16), la structure plate textile (16) étant réalisée de manière perméable aux liquides, la structure plate textile (16) présentant des fibres ayant un cœur de fibre et une enveloppe de fibre entourant entièrement le cœur de fibre, dans lequel le cœur de fibre présente un polymère de cœur de fibre et l'enveloppe de fibre présente un polymère d'enveloppe de fibre, le polymère de cœur de fibre ayant un point de fusion plus élevé que le polymère d'enveloppe de fibre, le point de fusion du polymère de cœur de fibre étant supérieur d'au moins 30 °C au point de fusion du polymère d'enveloppe de fibre, et
- une aide à l'introduction (21) pour introduire la pluralité de fibres creuses (12) enroulées dans la structure plate textile (16) dans un boîtier (14) du dialyseur (10).

13. Procédé de fabrication d'un dialyseur (10) selon l'une quelconque des revendications 1 à 11, présentant les étapes suivantes consistant à :
- enrouler une pluralité de fibres creuses (12) dans une structure plate textile (16), la structure plate textile (16) étant réalisée de manière perméable aux liquides, la structure plate textile (16) présentant des fibres ayant un cœur de fibre et une enveloppe de fibre entourant entièrement le cœur de fibre, dans lequel le cœur de fibre présente un polymère de cœur de fibre et l'enveloppe de fibre présente un polymère d'enveloppe de fibre, le polymère de cœur de fibre ayant un point de fusion plus élevé que le polymère d'enveloppe de fibre, le point de fusion du polymère de cœur de fibre étant supérieur d'au moins 30 °C au point de fusion du polymère d'enveloppe de fibre, et
- introduire la pluralité de fibres creuses (12) enroulées dans la structure plate textile (16) dans un boîtier (14) du dialyseur (10).
